# EUROPEAN PATENT SPECIFICATION

(11) **EP 0 420 932 B1**
(45) Date of publication and mention of the grant of the patent: **30.11.1994**
(21) Application number: 89907962.8
(22) Date of filing: 22.06.1989
(51) Int. Cl.: C07C 17/00, C07C 22/00

(54) **CATALYZED HYDROFLUORINATION OF ALKENES**
KATALYTISCHE HYDROFLUORIERUNG VON ALKENEN
HYDROFLUORATION CATALYSEE D'ALKYLENES

(30) Priority: 23.06.1988 US 210554
(43) Date of publication of application: 10.04.1991
(73) Proprietor: E.I. DU PONT DE NEMOURS AND COMPANY, Wilmington Delaware 19898 (US)
(72) Inventor: RAO, V., N., Mallikarjuna, Wilmington, DE 19809 (US)
(74) Representative: Woodcraft, David Charles
(86) International application number: US8902669
(87) International publication number: WO8912614

(56) References cited:
- US-A- 4 258 225

## Description

Process for the preparation of fluorinated alkanes by contacting alkenes, preferably halogenated alkenes, with hydrogen fluoride in the presence of TaCl₅ or TaBr₅.

### BACKGROUND OF THE INVENTION

A. E. Feiring, Journal of Fluorine Chemistry, 13, 7-18 (1979) discloses the use of tantalum pentafluoride as a catalyst for the addition of hydrogen fluoride to tetra- and trichloroethene and related compounds. The catalyst is also useful for fluorine chlorine exchange reactions. However, under the conditions of the batch experiments, catalysts such as BF₃, TaCl₅, Ta₂O₅, CoF₃' V₂O₅, ZrCl₄, NbCl₅, HgO, and WCl₆ showed no catalytic activity at 150°C for the addition of HF to tetrachloroethene.

The use of tantalum pentafluoride as a catalyst for the addition of hydrogen fluoride to unsaturated compounds has been disclosed and claimed in U.S. 4,258,225.

### SUMMARY OF THE INVENTION

This invention is a process for the preparation of fluorinated alkanes by contacting, at a temperature of O°C to 185°C under substantially anhydrous conditions, one molar equivalent of an alkene, preferably a halogenated alkene, selected from alkenes of the following formulas

R¹R²C=CR³R⁴ and R⁵R⁶C=CR⁷R⁸

wherein
R¹, R², R³, R⁴ and R⁵, R⁶, R⁷, R⁸ are each selected from the group represented by CₓZ₂ₓ+1, wherein Z is H, F, Br or Cl and wherein x = 0 to 10; and
at least one of the pairs R⁵ and R⁶, R⁷ and R⁸, R⁵ and R⁷, and R⁶ and R⁸ taken together is -(CH₂)ₙ- wherein "n" is an integer from 2 to 7 with the proviso that when R⁵ and R⁷ and/or R⁶ and R⁸ are trans, "n" must be 6 to 7, and when only two of the group R⁵, R⁶, R⁷, R⁸ are combined to form a cyclic structure the remaining two groups may be CₓZ₂ₓ₊₁, wherein Z is H, F, Br or Cl and x = 0 to 10,
with HF in the presence of at least one catalyst selected from tantalum pentachloride and tantalum pentabromide to produce reaction products; removing said reaction products from contact with said catalyst and isolating a fluorinated alkene having one or more fluorine atoms above the number present in the alkene.

### DETAILS OF THE INVENTION

The resulting fluorinated alkane produced in accordance with the invention has one hydrogen atom over and above the number of hydrogen atoms originally present in the alkene, and one or more fluorine atoms over and above the number of fluorine atoms originally present in the alkene.

The alkene starting materials of the invention do not substantially react with hydrogen fluoride alone under the conditions of temperature and pressure used in this invention and require the presence of added catalyst, specifically tantalum pentachloride (TaCl₅) or tantalum pentabromide (TaBr₅).

It is preferred that the TaCl₅ or TaBr₅ be used in an amount from 0.001 to 5 moles, preferably from 0.001 to 0.250 mole, per mole of starting alkene for reasons of economy and effectiveness. The catalyst is a commercially available crystalline solid and can be used alone or on a support, such as carbon.

The preferred halogenated alkenes are where at least two, and more preferably at least three, of R¹, R², R³ or R⁴ are Cl. Where at least one of R¹, R², R³ or R⁴ is CₓZ₂ₓ₊₁, it is preferred that x is 1 to 3 and, more preferably x is 1. The specifically preferred alkenes are Cl₂C=CCl₂, HClC=CCl₂, HFC=CF₂, ClFC=CF₂, H₂C=CCl₂, H₂C=CF₂, and H₂C=CHCl.

The reaction can be carried out in the liquid phase or vapor phase and at autogenous pressures or under constant pressure ranging from atmospheric to superatmospheric. Both the liquid phase and vapor phase processes include batch, semicontinuous and continuous modes of operation.

The reaction can be carried out at from O°C to 185°C. The preferred temperature is 35°C to 175°C.

Anhydrous or substantially anhydrous conditions means that water, which is detrimental to the reaction, should be excluded as much as possible from the reaction zone. The HF which is commercially available as anhydrous grade can be used in the reaction directly. Exclusion of moisture from the reaction vessel by means of appropriate moisture traps or other means is a routine procedure and is well known in the art.

It is preferred that 1 to 30 molar equivalents of HF be utilized, and more preferably from 3 to 30 molar equivalents of HF. At least 5 molar equivalents of HF is preferred, particularly for highly chlorinated alkenes such as Cl₂C=CCl₂. In practice from 15 to 30 molar equivalents of HF ensures the best combination of economics and fluorination yield.

The reaction vessel is constructed from materials which are resistant to the action of hydrogen halide such as nickel alloys, including monel, "Hastelloy" and "Inconel".

The liquid phase reactions are conducted by introducing the reagents in any order into the reaction vessel. Generally, the TaCl₅ or TaBr₅ and starting alkene are placed in the reaction vessel which is then cooled, and the required amount of hydrogen fluoride is condensed in the vessel. The vessel may be cooled in Dry Ice or liquid nitrogen and evacuated prior to the introduction of hydrogen fluoride to facilitate addition of the hydrogen fluoride. The contents of the vessel are raised to the appropriate reaction temperature and agitated by shaking or stirring for a length of time sufficient to cause the reaction to occur.

For liquid phase reactions, the amount of TaCl₅ or TaBr₅ used is from 0.001 to 5 moles per mole of starting alkene, preferably from 0.001 to 0.250 mole, more preferably from 0.005 to 1 mole per mole of starting alkene but most preferably from 0.01 to 0.5 mole per mole of staring alkene. In general, when one uses the higher molar concentration of the catalyst, he can use a lower molar proportion of the HF reactant. The preferred catalyst is TaCl₅. The amount of HF used in the reaction is from 1 to 30 molar equivalents per mole of organic starting material. The reaction can be carried out at from about O°C to 185°C. The preferred temperature is about 35°C to 175°C. Reaction time can be from 0.5 to 18 hours; the preferred times are from 1 to 8 hours.

In the vapor phase reaction, the reactants are introduced into the reactor above their boiling points. The temperature of the reactor must also be sufficient to keep the products of the reaction in the vapor state so that they pass over into a cooled receiver beyond the reactor rather than remain in the catalyst zone for a prolonged period of time.

For vapor phase reactions, it is convenient to support the TaCl₅ or TaBr₅ on an inert porous material such as carbon or other known supports. The preferred catalyst is TaCl₅. The amount of catalyst to inert support is from 10% to 50% by weight with amounts of about 25% being preferred. The amount of HF used in the reaction is from 1 to 30 molar equivalents per mole of organic starting material. The reaction can be carried out at from 50°C to 185°C. The preferred temperature is 70°C to 175°C. The contact time of the reagents with the catalyst nay be specified instead of reaction time. The combined operations of feed rate, control of reactor temperature and pressure and rate of removal of product from the reactor influence the residence time of the product in the reactor. It may be desirable to shorten the residence time for a given product within the reactor to control the formation of undesired products. Contact time is the average time that the reactant product mixture is in contact with the catalyst. Broadly, contact times of from 0.1 to 25 seconds are useful with preferred contact times in the range of 1 to 10 seconds.

Under the reaction conditions set forth above, a portion of the TaCl₅ or TaBr₅ may undergo fluorination, so that a portion of the TaCl₅ or TaBr₅ may be in the form of TaCl₅₋ₓ,Fₓ or TaBr₅₋ₓFₓ where "x" may be from 0 to about 5. The instant invention is understood to include that condition when it may exist.

Pressure is not critical. Atmospheric, superatmospheric and autogeneous pressures are the most convenient and are therefore preferred.

The fluorinated alkanes produced by the invention have utility as refrigerants, solvents and blowing agents.

### EXAMPLES

### General Experimental Procedure

The reactor consisted of a 100 ml high pressure cylinder made of monel or "Inconel" containing a magnetic stirrer and an internal thermocouple. Mounted on top of the reactor was a condenser and a back pressure regulator connected to an optional on line analytical system. Suitable inlet and exit lines were also present to allow for admission of reactants and withdrawal of products.

To the reactor was charged TaCl₅ in the desired amount. The reactor was then cooled and evacuated. The alkene starting material and the required amount of HF were then admitted to the reactor. The reactor was then pressurized with nitrogen to the desired pressure while still cold and then gradually brought to the desired operating temperature with stirring by using external heat provided with an oil bath. The back pressure regulator was set to the desired operating pressure prior to heating the reactor.

At the completion of the reaction, the product was isolated by conventional means and analyzed by gas chromatography. All the percentages reported in the Examples are area %.

### EXAMPLE 1

The General Experimental Procedure was followed using 16.5 g of tetrachloroethylene, 0.45 g of tantalum pentachloride and 15g of anhydrous HF. The reactor was brought to atmospheric pressure with nitrogen. The back pressure regulator was set at 13.6 bar (200 psig). The contents were heated and stirred at 119-122°C for about four hours. Analysis indicated 43.4% of CClF₂CHCl₂, 12.1% CCl₂FCHCl₂, and 30.1% unreacted starting material as the major products.

### EXAMPLE 2

The General Experimental Procedure was followed using 8.3 g of tetrachloroethylene, 2.0 g of tantalum pentachloride and 15 g of anhydrous HF. The reactor was pressurized to 13.6 bar (200 psig) when cold with nitrogen and the back pressure regulator was set for 27.2 bar (400 psig). The contents were heated and stirred at 135-138°C for two hours. Product analysis indicated 23% CF₃CHCl₂ and 68.9% CClF₂CHCl₂ as the major products.

### EXAMPLE 3

The General Experimental Procedure was followed using 16.5 g of tetrachloroethylene, 4.0 g of tantalum pentachloride and 15 g of anhydrous HF. The reactor was pressurized to 13.6 bar (200 psig) with nitrogen and the back pressure regulator was set for 34.6 bar (500 psig). The contents were heated and stirred at 142-144°C for about one hour. Analysis indicated 69.5% CF₃CHCl₂, and 27.4% CClF₂CHCl₂ as the major products.

### EXAMPLE 4

Example 3 was repeated with the exception that the reaction was run for 90 minutes. Analysis indicated 88.2% CF₃CHCl₂ and 9.5% CClF₂CHCl₂ as the major products.

### EXAMPLE 5

The General Experimental Procedure was followed using 22.4 g of trichloroethylene, 1.0 g of tantalum pentachloride and 10 g of anhydrous HF. The reactor was pressurized to 13.6 bar (200 psig) with nitrogen when cold and the back pressure regulator was also set for 13.6 bar (200 psig). The contents were gradually heated to 85-90°C with stirring and maintained at this temperature range for about one hour. Product analysis indicated the presence of 5.3% CF₃CH₂Cl, 73.0% CClF₂CH₂Cl, 14.9% CCl₂FCH₂Cl and 5.3% starting material in addition to minor amounts of other organics.

### EXAMPLE 6

Example 5 was repeated except that 15 g of anhydrous HF was used and the internal temperature was maintained at 78-80°C for about two hours with the back pressure regulator set at 34 bar (500 psig). Product analysis indicated 18.5% CF₃CH₂Cl, 75.4% CClF₂CH₂Cl and 5.1% CCl₂FCH₂Cl in addition to small amounts of other organics.

### EXAMPLE 7

The General Experimental Procedure was followed using 9.0 g of tetrachloroethylene, 3.0 g of tantalum pentachloride and 7.5 g of anhydrous HF. The reactor was pressurized to 13.6 bar (200 psig) when cold with nitrogen and the back pressure regulator was set for 34 bar (500 psig). The contents were heated with stirring at 144-147°C for two hours. Product analysis indicated 66% CF₃CHCl₂' 32.7% CClF₂CHCl₂, and small amounts of other organics.

## Claims

1. A process for the preparation of fluorinated alkanes by contacting, at a temperature of 0^{o}C to 185^{o}C, under substantially anhydrous conditions, one molar equivalent of an alkene selected from alkenes of the following formulas
R¹R²C=CR³R⁴ and R⁵R⁶C=CR⁷R⁸
wherein
R¹, R², R³, R⁴ and R⁵, R⁶, R⁷, R⁸ are each selected from the group represented by CₓZ₂ₓ+1, wherein Z is H, F, Br or Cl and wherein x = 0 to 10; and
at least one of the pairs R⁵ and R⁶, R⁷ and R⁸, R⁵ and R⁷, and R⁶ and R⁸ taken together is -(CH₂)ₙ- wherein "n" is an integer from 2 to 7 with the proviso that when R⁵ and R⁷ and/or R⁶ and R⁸ are trans, "n" must be 6 to 7, and when only two of the group R⁵, R⁶, R⁷, R⁸ are combined to form a cyclic structure the remaining two groups may be CₓZ₂ₓ₊₁, wherein Z is H, F, Br or Cl and x = 0 to 10,
with HF in the presence of at least one catalyst selected from tantalum pentachloride and tantalum pentabromide to produce reaction products; removing said reaction products from contact with said catalyst and isolating a fluorinated alkane having one or more fluorine atoms above the number present in the alkene.

2. A process for the preparation of fluorinated alkanes as claimed in claim 1 wherein at least one of R¹_{,} R², R³ and R⁴ and in at least one of R⁵, R⁶, R⁷ and R⁸, Z is F, Br or Cl.

3. The process of claim 2 wherein the amount of HF is 1 to 30 molar equivalents.

4. The process of claim 2 wherein the catalyst is present in an amount of 0.001 to 5 molar equivalents.

5. The process of claim 2 wherein the catalyst is tantalum pentachloride.

6. The process of claim 2 wherein the temperature is 35^{o}C to 175^{o}C.

7. The process of Claim 2 wherein at least two of R¹, R², R³ or R⁴ are Cl.

8. The process of claim 2 wherein at least three of R¹, R², R³ or R⁴ are Cl.

9. The process of claim 2 wherein x = 1 - 3.

10. The process of claim 2 wherein x = 1.

11. The process of claim 2 wherein the amount of HF is 3-30 molar equivalents.

## Patentansprüche

1. Verfahren zur Herstellung von fluorierten Alkanen durch Zusammenbringen bei einer Temperatur von 0 °C bis 185 °C unter im wesentlichen wasserfreien Bedingungen von 1 Moläquivalent eines Alkens, ausgewählt aus Alkenen der folgenden Formeln:
R¹R²C=CR³R⁴ und R⁵R⁶C=CR⁷R⁸
worin
R¹, R², R³, R⁴ und R⁵, R⁶, R⁷, R⁸ jeweils ausgewählt werden aus der Gruppe, dargestellt durch CₓZ₂ₓ₊₁, worin Z für H, F, Br oder Cl steht, und worin x = 0 bis 10, und wenigstens eines der Paare R⁵ und R⁶, R⁷ und R⁸, R⁵ und R⁷ und R⁶ und R⁸ zusammengenommen für -(CH₂)ₙ- stehen, worin "n" eine ganze Zahl von 2 bis 7 bedeutet, mit der Maßgabe, daß, wenn R⁵ und R⁷ und/oder R⁶ und R⁸ in trans-Stellung stehen, "n" 6 bis 7 bedeuten muß, und wenn nur zwei der Gruppe R⁵, R⁶, R⁷, R⁸ unter Bildung einer cyclischen Struktur kombiniert werden, die restlichen zwei Gruppen CₓZ₂ₓ₊₁ sein können, worin Z für H, F, Br oder Cl steht, und x = 0 bis 10,
mit HF in Gegenwart von wenigstens einem Katalysator, ausgewählt aus Tantalpentachlorid und Tantalpentabromid, um die Reaktionsprodukte zu erzeugen, Entfernen der genannten Reaktionsprodukte von dem Kontakt mit dem genannten Katalysator und Isolieren eines fluorierten Alkans mit einem oder mehreren Fluoratomen mehr als die in dem Alken vorhandene Anzahl.

2. Verfahren zur Herstellung von fluorierten Alkanen nach Anspruch 1, bei dem in wenigstens einem von R¹, R², R³ und R⁴ und in wenigstens einem von R⁵, R⁶, R⁷ und R⁸ Z für F, Br oder Cl steht.

3. Verfahren nach Anspruch 2, bei dem die Menge von HF 1 bis 30 Moläquivalente beträgt.

4. Verfahren nach Anspruch 2, bei dem der Katalysator in einer Menge von 0,001 bis 5 Moläquivalenten vorhanden ist.

5. Verfahren nach Anspruch 2, bei dem der Katalysator Tantalpentachlorid ist.

6. Verfahren nach Anspruch 2, bei dem die Temperatur 35 °C bis 175 °C beträgt.

7. Verfahren nach Anspruch 2, bei dem wenigstens zwei von R¹, R², R³ oder R⁴ für Cl stehen.

8. Verfahren nach Anspruch 2, bei dem wenigstens drei von R¹, R², R³ oder R⁴ für Cl stehen.

9. Verfahren nach Anspruch 2, bei dem x = 1 - 3.

10. Verfahren nach Anspruch 2, bei dem x = 1.

11. Verfahren nach Anspruch 2, bei dem die Menge von HF 3 - 30 Moläquivalente beträgt.

## Revendications

1. Procédé pour la préparation d'alcanes fluorés en mettant en contact, à une température de 0 °C à 185 °C, dans des conditions substantiellement anhydres, un équivalent molaire d'un alcène choisi parmi les alcènes des formules suivantes:
R¹R²C=CR³R⁴ et R⁵R⁶C=CR⁷R⁸
où R¹, R², R³, R⁴ et R⁵, R⁶, R⁷, R⁸ sont chacun choisis dans le groupe représenté par CₓZ₂ₓ₊₁, où Z est H, F, Br ou Cl et où x = 0 à 10 ; et au moins l'une des paires R⁵ et R⁶, R⁷ et R⁸, R⁵ et R⁷, et R⁶ et R⁸ pris ensemble est -(CH₂)ₙ, où "n" est un nombre entier de 2 à 7, avec la condition que lorsque R⁵ et R⁷ et/ou R⁶ et R⁸ sont trans, "n" doit être 6 à 7, et lorsque seulement deux des groupes R⁵, R⁶, R⁷, R⁸ sont combinés pour former une structure cyclique, les deux groupes restants peuvent être CₓZ₂ₓ₊₁, où Z est H, F, Br ou Cl et x = 0 à 10, avec HF, en présence d'au moins un catalyseur choisi parmi le pentachlorure de tantale et le pentabromure de tantale, pour obtenir des produits de réaction ; en retirant lesdits produits de réaction du contact avec ledit catalyseur et en isolant un alcane fluoré ayant un ou plusieurs atomes de fluor de plus que le nombre présent dans l'alcène.

2. Procédé pour la préparation d'alcanes fluorés selon la revendication 1, dans lequel au moins l'un de R¹, R², R³ et R⁴ et au moins l'un de R⁵, R⁶, R⁷ et R⁸, Z est F, Br ou Cl.

3. Procédé selon la revendication 2, dans lequel la quantité de HF est 1 à 30 équivalents molaires.

4. Procédé selon la revendication 2, dans lequel le catalyseur est présent en une quantité de 0,001 à 5 équivalents molaires.

5. Procédé selon la revendication 2, dans lequel le catalyseur est le pentachlorure de tantale.

6. Procédé selon la revendication 2, dans lequel la température est 35 °C à 175 °C.

7. Procédé selon la revendication 2, dans lequel au moins deux de R¹, R², R³ et R⁴ sont Cl.

8. Procédé selon la revendication 2, dans lequel au moins trois de R¹, R², R³ et R⁴ sont Cl.

9. Procédé selon la revendication 2, dans lequel x = 1 à 3.

10. Procédé selon la revendication 2, dans lequel x = 1.

11. Procédé selon la revendication 2, dans lequel la quantité de HF est 3 à 30 équivalents molaires.
